# EUROPEAN PATENT APPLICATION

(11) **EP 2 277 443 A1**
(43) Date of publication of application: **26.01.2011**
(21) Application number: 09460032.7
(22) Date of filing: 22.07.2009
(51) Int. Cl.: A61B 5/16, G06F 19/00

(54) **Multisensory education system**

(71) Applicant: Slawinski, Andrezj, Dr., 7243 Pany (CH)
(72) Inventor: Slawinski, Andrezj, Dr., 7243 Pany (CH)
(74) Representative: Pomianek, Grazyna

(57) **Abstract**

A multisensory education system to be used especially in education, interpersonal mental trainings, in the SPA sector (Wellness), as well as in the individual use. The essence of this invention consists in integrating multisensory stimulation systems with biofeedback and psychoactivity techniques. Said system comprises the low frequency vibration elements (1-8) transferring their vibrations having different frequencies directly to a user to generate interference waves in the user's body.

A unit optically simulating skin, especially a high resolution laser scanner (RGB) or other light sources (23) with a low rest light emission (LEDs) can stimulate the human body to emit enough light to be measured by real time enhanced cameras or photomultipliers, creating in this way a psychophysiological map of the human body. Multimedia in High Definition give fantastic vision sharpness and surround sound.

## Description

This invention relates to a multisensory education system to be used especially in education, interpersonal mental trainings, in the SPA sector (Wellness) , as well as in the individual use.

Technologies using biofeedback methods are known designed for controlling software modules of individual persons, as well as modules of some users connected to control one or more common parameters.

In the specification WO 03/090176 a multimedia system for human sensory education and assessment is disclosed. That system relates to software and hardware-assisted systems that provide educational content, simulation of certain human sensory conditions, and a personal assessment of one or more of a number of human sensory systems. This system comprises an administrative subsystem and a delivery device, said administrative subsystem downloading to said delivery device at least one suitable content module associated with one or more human senses, and the delivery device configures downloaded content modules to enable a user to study educational modules and perform sensory tests and assessments, and moreover it provides result data to said administrative subsystem for storing, reporting, and retrieving later by authorized persons.

It is an object of this invention to further improve said biofeedback method so as to enable using it in education, training, wellness sector, SPA or the like.

The essence of this invention consists in integrating multisensory stimulation systems with biofeedback and psychoactivity techniques.

A multisensory education system according to the invention comprises at least one sensor gathering psychophysiological parameters from any user, especially by measuring his/her GSR (skin resistivity), EEG, EMG, EKG and the like, at least two mutually independently controlled low frequency vibration elements, what makes possible to create interference waves in a user's body, signals from said waves being fed to a multichannel source and a main controlling and processing unit comprising a suitable software where they are converted into active data and are depicted in a 2D or 3D picture by a graphic display, especially a monitor, projector, display glasses or the like, in order to create interactive connection between active data shown in a display and user's sense organs and sensors registering changes in psychophysiological states.

Said vibration elements are independently controlled by a multichannel system comprising amplifiers and filters having a multichannel connection with a multichannel source and a main controlling and processing unit. Vibrations created by the interference effect are registered by one or more sensors from which signals are transmitted to said multichannel source and main controlling and processing unit.

The system comprises an additional unit stimulating optically a skin, said unit comprising especially a series of LEDs (RGB) or a laser scanner controlled by said multichannel source and main controlling and processing unit.

The ultraweak photon emission (UPE) created in such a way is measured especially by means of photomultipliers or suitable CCD cameras, or the like, wherein measured values are led to the multichannel source and main controlling and processing unit.

The system according to the invention comprises additionally a multichannel sound amplifying arrangement controlled by said multichannel system of power amplifiers and filters, said arrangement being formed especially by loudspeakers or multichannel (surround) earphones, as well as an optical stimulation arrangement comprising especially a series ofLEDs (RGB) controlled by said multichannel source and main controlling and processing unit.

Said multichannel source and main controlling and processing unit comprises a HID device (Human Input Device or Human Interface Device) in order to input user's data especially by means of a keyboard, joystick, mouse, track-ball, or the like, and it has a multichannel A/D interface for sensors gathering psychophysiological parameters and sensors measuring created vibrations.

Additionally the multichannel source and main controlling and processing unit is provided with a microphone or microphones for verbal communication, as well as with a camera or cameras for visual communication.

In the multichannel source and main controlling and processing unit signals in form of active data from one or more users can be converted, said unit comprising in such a case a network system for transmitting data between a central and individual users.

Said multichannel source and main controlling and processing unit can be constructed as a server, also as an internet server.

Psychophysiological and vibration parameters can be transmitted by means of internet, intranet, data transmission or wireless.

A coordinator may be provided in this system, said coordinator changing psychophysiological parameters of individual users by handling group data.

A method of using the sensory education system according to the invention not in a therapy of human or animal organisms during a session with one or more users consists in registering by means of sensors psychophysiological data from one or more users, registering vibrations of generated interferences in result of low frequency vibrations having different frequency values, received from at least two elements and transferred directly to a user, converting and calculating active data attributed to any user and/or to a group of users in said multichannel source and main controlling and processing unit, as well as in presenting said data attributed to any user or a group of users in a graphic display system.

Additionally a measured value of ultraweak photon emission created in result of optical stimulation of user's skin is registered and fed to the multichannel source and main controlling and processing unit in order to recalculate it into active data and to display it by means of a graphic display.

Active data are shown by linear presentation of psychophysiological states and by nonlinear interactions with one or more users.

A session run may be varied by individual and/or group reactions, as well as by coach's interventions in relation to individual users or to a whole group.

To communicate with the system an HID element, psychophysiological data sensors, graphic display and sound amplifying system are provided.

The multisensory education system according to the invention incorporates the State of Art Technology concerning learning, mental training, art experience and entertainment. High-Definition Multimedia warrant fantastic vision sharpness and surround sound.

Additional vibration stimulation of independent elements generating interference waves causes "inner massage" in a user's body, and in result of that said user feels waves or rotations. Registration of created vibrations by means of sensors and evaluating said vibrations by the multi channel source and main controlling and processing unit enables projecting interference effects in selected parts of a user's body.

A high resolution RGB laser scanner or other light sources with low rest light emission (LEDs) can stimulate human body to emit enough light to be measured by real time enhanced cameras or photomultipliers, giving in this way a psychophysiological map of a human body. This mapping can be used for evaluation and for real time modification by the user - a unique learning process by controlling his/her own psychophysiology. Different stimulation colors can be used in the psychointeractive process individually or in groups and can be treated as another psychophysiological parameter such as GSR or EEG.

Light stimulation (Audio Strobe) by means of a chain of LEDs (RBG) provides a user with pleasant mind states.

The invention is disclosed in its embodiment shown in the drawing in which Fig. 1 presents a circuit diagram of the system according to the invention, and Fig. 2 and 3 present schematically a method of using this system.

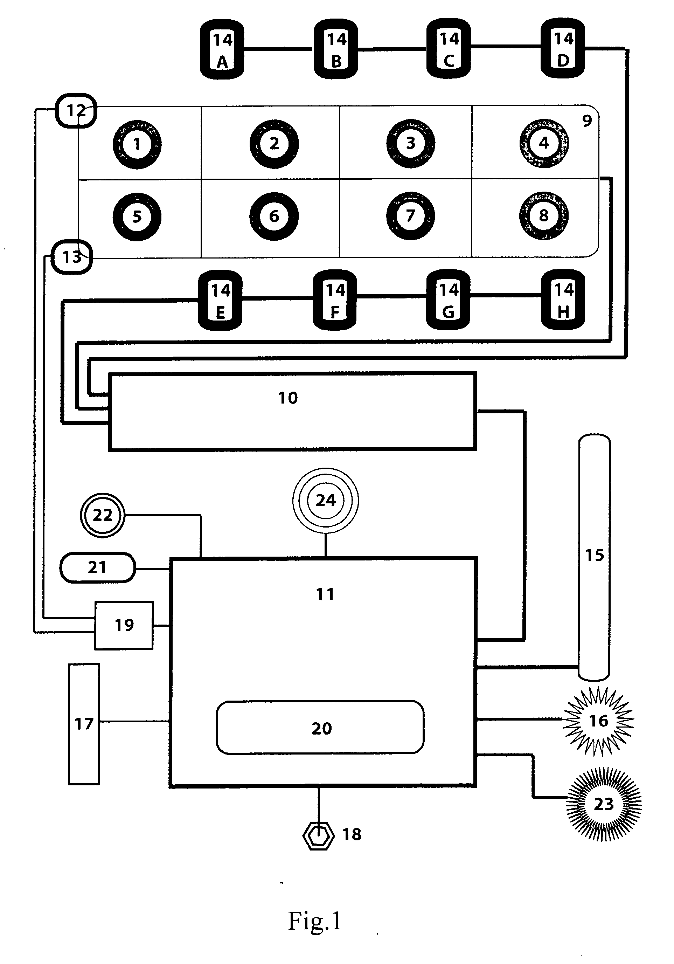

The system shown in Fig. 1 comprises for example eight independent low frequency vibration elements 1- 8. These elements are assembled on a mattress, chair, hammock chair 9, or the like in such a way that they transfer vibrations directly onto a user. When for example vibration frequencies of elements 1 and 5 are 50 Hz and 55 Hz, interference vibration (rumble) is created the frequency of which amounts 5 Hz.

An intelligent software of individual vibration elements 1 - 8 causes an "inner massage" during which a user feels waves or rotation . Vibrations are measured by sensors 13. The system is provided with a set of sensors 12 gathering psychophysiological parameters of a user, in this number GSR (skin resistivity), EEG, EMG, EKG and the like. Signals from sensors 12 and 13 are transferred to the multichannel source and main controlling and processing unit 11 provided with the suitable software 20, where they are converted into an active data form.

The system comprises the unit 23 stimulating optically user's skin, said unit comprising especially a series of LEDs (RGB) or a laser scanner (RGB) and is controlled by means of said multichannel source and main controlling and processing unit 11. The created ultraweak photon emission is measured by the unit 23 comprising especially a photomultiplier or photomultipliers, or suitable CCD cameras, or the like. Said multichannel source and main controlling and processing unit 11 comprises a multichannel source for the multichannel power amplifier and filters for controlling vibration levels of the elements 1 - 8, as well as a sound level in the sound amplifying elements 14A - 14H, especially loudspeakers or multichannel earphones.

Active data from the multichannel source and main controlling and processing unit 11 are visualized in the graphic display unit 15 as a D2 or D3 picture in order to create an interactive connection between those data and sense organs of a user and sensors registering changes of psychophysiological states.

The graphic display unit 15 comprises a monitor or display glasses, or the like. Additionally said system comprises the optical stimulation unit 16 controlled by the multichannel source and main controlling and processing unit 11 which especially comprises a series of LEDs (RGB). User's data are introduced into the system by the HID unit 17 that may be used in form of a keyboard, joystick, mouse, track ball, or the like.

The system is provided with the A/D multichannel interface 19 for the sensors 12 acquiring psychophysiological parameters, and for the sensors 13 measuring generated vibrations.

Verbal communication can be carried out by means of the microphone or microphones 21, and visual communication - by means of the camera or cameras 22 coupled with the multichannel source and main controlling and processing unit 11.

The system may be adapted for serving simultaneously a number of users. It is provided for the data transmission between a central and individual users. The data transmission between a central and individual users is realized by network system 18 connected with the multichannel source and main controlling and processing units 11.

In one embodiment of this invention a personal computer is used as said multi channel source and main controlling and processing unit 11, and in another embodiment a server, especially an internet server, is used as said unit 11.

In one embodiment psychophysiological and vibration parameters are transferred by means of internet, and in another - by intranet, and in still another by the data transmission or wireless.

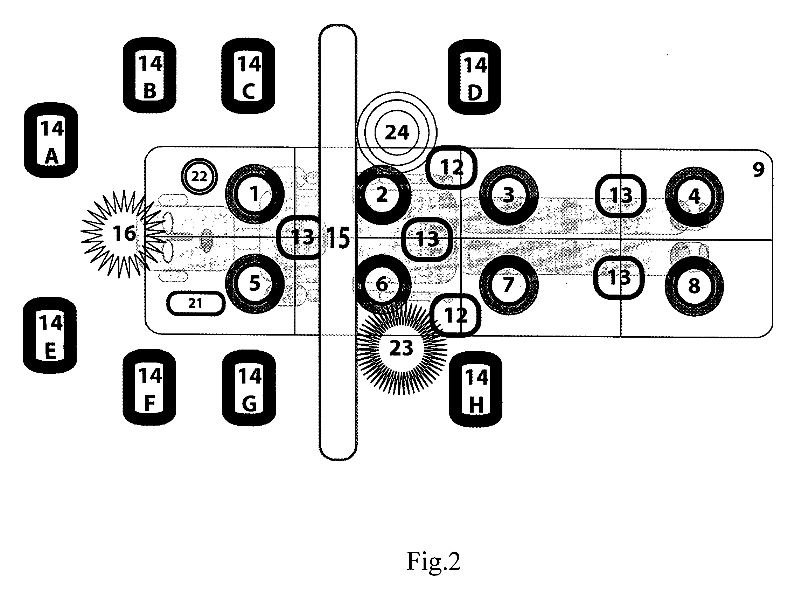

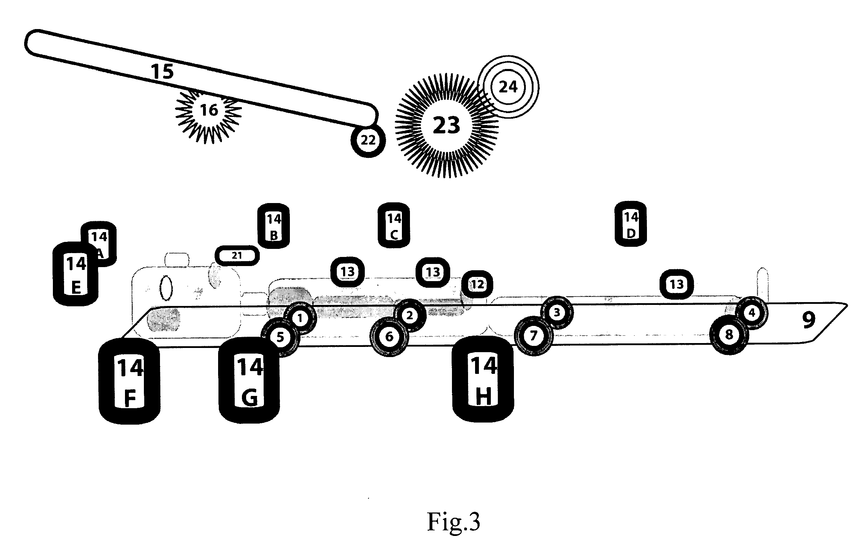

As it is shown in Fig. 2 and Fig. 3, a user is laying down onto the element 9, e.g. a mattress, and is wearing the sensors 12 and 13. He/she confirms by means of the HID interface 17 his/her identity and readiness to carry out the session. The session is started by an operator or by a server or a user depending on whether the system is configured in the web mode or as the individual local one. The course of a selected session is adjusted in the real time to psychophysiological user's reactions. Interactive connections between psychophysiological data and user's sense organs, as well as sensors registering changes of psychophysiological states are created not only by linearly displaying psychophysiological states, but also by nonlinear interactions with a user or in a network with other users. The real use is planned in telecoaching, where a coach can meet with a training person or a group of persons in the virtual space to carry out individual and/or group sessions. A course of a session can be changed by individual or group reactions, as well as by coach's/moderator's interventions in relation to some users or a whole group. In the last case one can speak about group psychointeractivity.

## Claims

1. A multisensory education system comprising at least one sensor (12) for gathering psychophysiological parameters of a user, said parameters being transferred to the multichannel source and main controlling and processing unit (11) provided with the suitable software (20), by means of which said parameters are converted into active data, as well as the graphic display unit (15) visualizing data converted in said multichannel source and main controlling and processing unit (11) in order to create an interactive connection between active data shown in said display unit and user's sense organs, as well as sensors registering changes of psychophysiological states, **characterized in that** it has at least two low frequency vibration elements (1-8) transferring their vibrations directly to a user, said elements being controlled independently by means of the multichannel system (10) of power amplifiers and filters, said system being coupled by many channels with the multichannel source and main controlling and processing unit (11) and comprises at least one sensor (13) for measuring created interference vibrations and transferring a signal to the multichannel source and main controlling and processing unit (11).

2. A system according to Claim 1, **characterized by** that it comprises the optical skin stimulation unit (23) controlled by the multichannel source and main controlling and processing unit (11), as well as the unit (24) measuring ultraweak photon emission (UPE) in result of said stimulation, a value of said emission being transferred to said multichannel source and main controlling and processing unit (11).

3. A system according to Claim 1 or 2, **characterized by** that it comprises the multichannel sound amplifying system (14A-14H) controlled by said unit (10) of multichannel power amplifiers and filters.

4. A system according to Claim 1 to 3, **characterized by** that it comprises the optical stimulation unit (16) controlled by said multichannel source and main controlling and processing unit (11).

5. A system according to Claim 1 to 4, **characterized by** that said multichannel source and main controlling and processing unit (11) comprises the HID unit (17) for introducing user's data.

6. A system according to Claim 1 to 5, **characterized by** that it comprises the multichannel A/D interface (19) for the sensors (12) in order to gather psychophysiological parameters, and for the sensors (13) measuring created vibrations.

7. A system according to Claim 1 to 6, **characterized by** that said multichannel source and main controlling and processing unit (11) is provided with the microphone or microphones (21) enabling the verbal communication.

8. A system according to Claim 1 to 7, **characterized by** that said multichannel source and main controlling and processing unit (11) is provided with the camera or cameras (22) enabling the visual communication.

9. A system according to Claim 1 to 8, **characterized by** that said multichannel source and main controlling and processing unit (11) converts and controls signals comprising active data received from a number of users.

10. A system according to Claim 9, **characterized by** that said multichannel source and main controlling and processing unit (11) is connected with the network unit (18) in order to transmit data between a central and particular users.

11. A system according to Claim 9 or 10, **characterized by** that a coordinator is provided changing psychophysiological parameters of individual users by manipulating with group data values.

12. A method of using a multisensory education system not in human or animal therapy in a session with one or more users consisting in registering psychophysiological data by means of the sensors (12), converting and calculating active data attributed to a user and/or a group of users in said multichannel source and main controlling and processing unit (11), as well as showing said data attributed to a user and/or a group of users in the graphic display unit (15), **characterized by** that from at least two low frequency vibration elements (1-8) transferring directly onto a user vibrations having different frequency values and creating interference waves the measured values of created vibrations are registered in the sensors (13) and are introduced into said multichannel source and main controlling and processing unit (11) in order to recalculate them into active data, as well as to show them in the graphic display unit (15).

13. A method according to Claim 12, **characterized in that** a measured value of ultraweak photon emission created by optically stimulating user's skin is additionally registered and introduced into said multichannel source and main controlling and processing unit (11) to recalculate it into active data, as well as to show it by means of the graphic display unit (15).

14. A method according to Claim 12 or 13, **characterized in that** active data are shown by the linear visualization of psychophysiological states, as well as by nonlinear interactions with a user or users.

15. A method according to Claim 12 or 13, **characterized in that** a course of a session may be changed by individual or group reactions, as well as by interventions of a coach or a coordinator in relation to individual users or to the whole group.
